(19) Europäisches Patentamt | European Patent Office | Office européen des brevets

(11) **EP 3 459 554 B1**

(12) **EUROPEAN PATENT SPECIFICATION**

(45) Date of publication and mention
of the grant of the patent:
**27.10.2021 Bulletin 2021/43**

(21) Application number: **17799375.5**

(22) Date of filing: **16.05.2017**

(51) Int Cl.:
*A61K 36/064* (2006.01)     *A61P 3/10* (2006.01)
*A61K 31/519* (2006.01)     *A61K 31/525* (2006.01)
*A61K 31/7076* (2006.01)     *A23L 33/145* (2016.01)
*A61K 38/06* (2006.01)

(86) International application number:
**PCT/JP2017/018358**

(87) International publication number:
**WO 2017/199951 (23.11.2017 Gazette 2017/47)**

(54) **YEAST EXTRACT HAVING DIABETES PREVENTION EFFECT**

HEFEEXTRAKT MIT DIABETESPRÄVENTIONSWIRKUNG

EXTRAIT DE LEVURE À EFFET PRÉVENTIF CONTRE LE DIABÈTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.05.2016 JP 2016099082**

(43) Date of publication of application:
**27.03.2019 Bulletin 2019/13**

(73) Proprietor: **KOHJIN Life Sciences Co., Ltd.
Tokyo 100-0006 (JP)**

(72) Inventors:
• **SAIKI, Tomomi
Saiki-shi
Oita 876-8580 (JP)**
• **HAMASAWA, Kazuhiro
Saiki-shi
Oita 876-8580 (JP)**

(74) Representative: **Müller-Boré & Partner
Patentanwälte PartG mbB
Friedenheimer Brücke 21
80639 München (DE)**

(56) References cited:
**WO-A1-01/19542        JP-A- 2009 291 076
JP-A- 2011 178 794      JP-A- 2016 037 485**

• **DATABASE WPI Week 201001 Thomson
Scientific, London, GB; AN 2009-S33499
XP002794648, & JP 2009 291076 A (KANEKA
CORP) 17 December 2009 (2009-12-17)**
• **DATABASE WPI Week 200707 Thomson
Scientific, London, GB; AN 2007-065308
XP002794649, & JP 2006 347975 A (BIO
SOLUTIONS KK) 28 December 2006 (2006-12-28)**
• **JACH M E ET AL: "Use of yeasts for prevention
and therapy", CURRENT ISSUES IN PHARMACY
AND MEDICAL SCIENCES, MEDICAL
UNIVERSITY OF LUBLIN, POL, vol. 26, no. 2, 1
January 2013 (2013-01-01), pages 198-202,
XP009516343, ISSN: 2300-6676, DOI:
10.12923/J.2084-980X/26.2/A.17**

**Description**

The present disclosure provides a food composition and a pharmaceutical composition each containing an aqueous solvent extract derived from torula yeast as an active ingredient. Specifically, the present disclosure provides a food composition and a pharmaceutical composition each having an excellent diabetes prevention effect.

The diabetes population continues to increase worldwide, and the number of people suffering from diabetes worldwide is 415 million as of 2015. Furthermore, people who are not diagnosed as diabetes, but who have pre-diabetes involving blood sugar levels higher than normal values are increasing rapidly. Along with this, the medical expenses related to diabetes now reach about 81 trillion yen (about NT $ 22 trillion) in the world, and is expected to increase in the future.

Diabetes is classified into insulin-dependent type I diabetes and insulin-independent type II diabetes. In particular, people suffering from type II diabetes that develops in an acquired manner due to environmental factors such as lifestyle habits are increasing, accounting for about 90% of diabetic patients. Since diabetes is a metabolic abnormality due to the persistence of a hyperglycemic state, it is likely to cause serious complications in organs of the whole body, such as eye, kidney, nervous system, vascular system, and skin, which becomes a serious problem.

As therapeutic agents for diabetes, insulin preparations, sulfonylurea preparations that promote secretion of insulin, $\alpha$-glucosidase inhibitors that delay digestion and absorption of carbohydrates, thiazolidine-based preparations that improve insulin resistance, and the like are known. However, these synthetic drugs are not easy to obtain because the prescription of a doctor is necessary, and additionally the effect is not sufficient in some cases. Furthermore, unless such drugs are taken strictly, there are various side effects such as a hypoglycemic state and a problem also exists in safety.

Therefore, it is important to control the blood sugar level even in a patient having a normal blood sugar level or a patient corresponding to pre-diabetes, and various food-derived compositions have been reported. For example, there are peptides derived from $\beta$-lactoglobulin hydrolyzates (Patent Literature 1), paramylon derived from euglena (Patent Literature 2), indigestible dextrins (Patent Literature 3), loquat seed extracts (Patent Literature 4), etc. Fermented products of Laminaria japonica by Bacillus natto (Patent Literature 5) have been reported as those derived from microorganisms.

On the other hand, a yeast extract extracted from yeast is an extract obtained through hot water extraction, enzyme treatment, self-digestion, etc. from yeast cultured or the like. The yeast extract contains an amino acid such as glutamic acid which is an umami ingredient and a nucleic acid, and thus is used as a food material. It is also known that the yeast extract is not only used as a food material but also has a biological control function. For example, it is known that the yeast extract obtained from yeast cultured under specific conditions controls the blood sugar level (Patent Literature 6).

Furthermore, Patent Literature 7 describes a yeast extract having vasorelaxing action.

Under such circumstances, a composition for controlling the blood sugar level, that is more effective, safe, and inexpensively available, is required.

Patent Literature 1: JP 2011-144167 A
Patent Literature 2: JP 2014-118374 A
Patent Literature 3: JP 2005-289847 A
Patent Literature 4: JP 2005-325029 A
Patent Literature 5: JP 2015-21000 A
Patent Literature 6: JP 2009-291076 A
Patent Literature 7: JP 2016-037485 A

An object of the present invention is to provide an agent for use in the prevention or treatment of diabetes by suppressing an increase in blood sugar level that is effective for diabetic patients, patients corresponding to pre-diabetes, and healthy subjects, has little side effects, and is safe. Furthermore, it is desirable that the agent for use in the prevention or treatment of diabetes by suppressing an increase in blood sugar level is food-derived and inexpensive, and can be used as a food, a pharmaceutical product or the like.

In the present invention, when the glucose uptake ability in rat myoblasts (L6 cells) was confirmed, it has been found that yeast extracts obtained by a simple procedure comprising treating yeast with hot water, an acid or an alkali, or an enzyme have a glucose uptake promoting action.

Furthermore, it has been found that oral administration of the yeast extract to diabetic model rats (GK rats) for a long term suppresses an increase in fasting blood sugar level.

The yeast extract which is an agent for use in the prevention or treatment of diabetes by suppressing an increase in blood sugar level according to the present invention is an extract extracted from yeast known as a food. Therefore, it can be relatively easily and inexpensively produced, and can also be continuously ingested safely as a food for a long term in order to improve diabetic patients, to prevent and improve diabetes in patients corresponding to pre-diabetes, and further to prevent diabetes in healthy people.

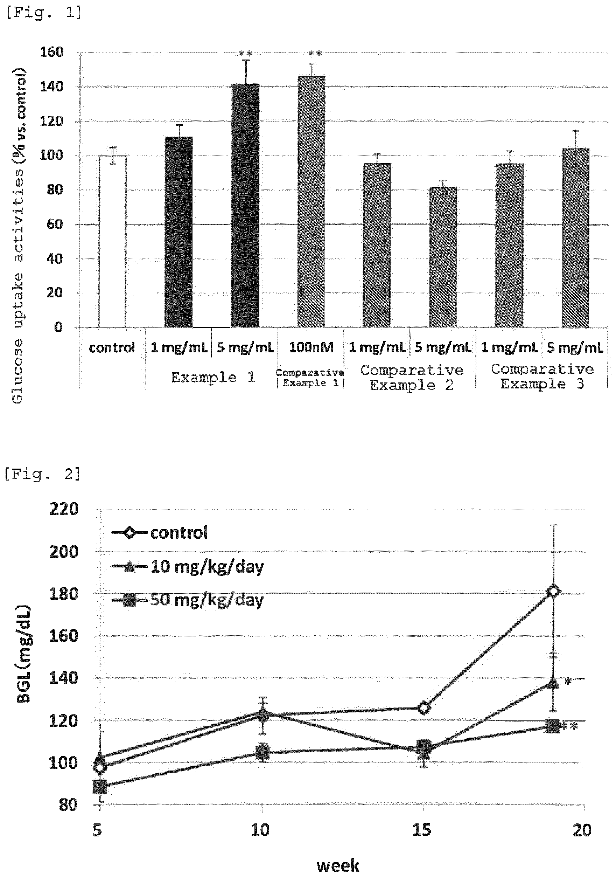

Fig. 1 shows glucose uptake activities (%) of Example 1, Comparative Example 1, Comparative Example 2, and Comparative Example 3 in Test 1. The "1 mg/mL" and "5 mg/mL" for Example 1 and Comparative Examples 2 and 3 indicate the concentrations of a sample solution. The "100 nM" for Comparative Example 1 indicates the final concentration of insulin. The symbol "*" indicates a significant difference at a risk rate of 5%, and the symbol "**" indicates a significant difference at a risk rate of 1%.

Fig. 2 shows a fasting blood sugar level (BGL) in Test 2. The "Week" indicates the age in week of GK rats. The symbol "*" indicates a significant difference at a risk rate of 5%, and the symbol "**" indicates a significant difference at a risk rate of 1%.

Any yeast can be used for producing the yeast extract of the present invention as long as it is a commonly-used yeast. Specific examples of the yeast include baker's yeast, beer yeast and torula yeast. Among them, torula yeast (Candida utilis) is particularly desirable.

The yeast extract used in the present invention is a yeast extract having a glutathione content of 15% by mass or more, an adenine content of 0.05% by mass or more, an adenosine content of 0.05% by mass or more, a folic acid content of 0.010% by mass or more, and a riboflavin content of 0.010% by mass or more. A yeast extract having a glutathione content of 15% by mass or more, an adenine content of 0.1% by mass or more, an adenosine content of 0.1% by mass or more, a folic acid content of 0.010% by mass or more, and a riboflavin content of 0.010% by mass or more is more desirable.

A yeast extract containing glutathione in an amount of 15% by mass or more can be obtained by extracting the yeast extract from yeast cells containing glutathione. There are no particular restrictions on the yeast culture form, but one of batch culture and continuous culture is generally used. Also, a commonly-used medium can be used. For example, glucose, acetic acid, ethanol, glycerol, molasses, sulphite pulp waste liquids and the like are used as a carbon source, and urea, ammonia, ammonium sulfate, ammonium chloride, nitrate and the like are used as a nitrogen source. As phosphoric acid, potassium, and magnesium sources, for example, lime perphosphate, ammonium phosphate, potassium chloride, potassium hydroxide, magnesium sulfate, magnesium chloride and the like can be used, and other inorganic salts such as zinc ions, copper ions, manganese ions, and iron ions are added. In addition, vitamins, amino acids, nucleic acid-related substances and the like may be added, or organic substances such as casein, yeast extract, meat extract, and peptone may be added. The culture temperature is 21 to 37°C, preferably 25 to 34°C, and the pH is 3.0 to 8.0, preferably 3.5 to 7.0.

After culturing yeast cells, the yeast extract of the present invention is extracted. The extraction method of the yeast extract is not particularly limited, but it is generally possible to carry out the extraction by a self-digestion method, a hot water extraction method, an enzyme extraction method, an acid or alkali extraction method, or a combination thereof.

When the yeast extract is extracted by self-digestion, for example, a yeast culture liquid or the like is stirred at 55°C for 4 hours. In the case of the enzyme extraction method, a cell wall digesting enzyme, protease or the like is added to a yeast culture liquid or the like and reacted therewith for extraction. In the acid extraction method, a yeast culture liquid or the like is adjusted to be acidic with sulfuric acid or the like for extraction. In the alkaline extraction method, a yeast culture liquid or the like is adjusted to be alkaline for extraction. Alternatively, a combination such as self-digestion followed by enzyme extraction is also possible.

After the yeast extract has been extracted, the yeast residue is separated by centrifugation or the like, concentrated, and then freeze dried or hot air dried, thereby making it possible to obtain a glutathione-containing yeast extract. Glutathione contained in the yeast extract of the present invention refers to reduced glutathione.

The yeast extract of the present invention contains glutathione in an amount of 15% by mass or more. In order that the glutathione content in the yeast extract is 15% by mass or more, it is desirable to use a cultured yeast having as high a glutathione content as possible. By extracting the yeast extract from such yeast cells by the above-mentioned extraction method, the product of the present invention can be efficiently produced. As a method for increasing the glutathione content in yeast, a known method may be used. For example, there are a method in which zinc ions are added to a medium (JP 2000-279164 A), a method for obtaining a yeast strain having a high glutathione content using cadmium resistance and macrolide antibiotic resistance as indexes (JP 2006-42637 A and JP 2006-42638 A), and the like. It can also be obtained by extraction from mutant yeast cells as disclosed in JP 2011-103789 A. In addition, generally-sold yeast extracts include "HITHION EXTRACT YH-15" manufactured by KOHJIN Life Sciences Co., Ltd.

In the present invention, the glutathione concentration was measured by the DTNB-HPLC method (Journal of Chromatography, 194 (1980) 424-428). The glutathione content of the present application refers to the reduced glutathione content.

The adenine and adenosine concentrations can be quantified by the following measuring method and conditions. Specifically, 20 mg of a sample is diluted to 100 mL with a 0.1w/w% formic acid solution and analyzed by liquid chromatography tandem mass spectrometry (LC-MS/MS). The reagents adenine and adenosine (each free) are used as standards and subjected to LC-MS/MS under the same conditions. Calibration curves are prepared based on the peak area, and the contents of adenine and adenosine in the sample are calculated.

The analysis conditions can be set as follows.

<LC conditions>

- Column: Inertsil ODS-3 (2.1 mm × 150 mm)
- Mobile phase: A; 0.1w/w% formic acid, B; 0.1w/w% formic acid/acetonitrile, A → B; 20 min linear gradient
- Flow rate: 0.2 mL/min
- Column temperature: 45°C

<MS/MS conditions>

- Measuring equipment: amazon (Bruker daltonics)
- Ionization method: ESI-Positive
- Monitor ion: adenine; m/z 136 → 136, adenosine; m/z 268 → 136

The concentrations of riboflavin and folic acid were measured according to the analysis method prescribed in the Standard Tables of Food Composition in Japan. For measurement of the NAD (nicotinamide adenine dinucleotide) concentration, information known by those skilled in the art can be used, but quantification can be carried out, for example, by the following measuring method and conditions. Specifically, 0.1g of a sample is diluted to 100 mL with distilled water and then filtered through a 0.45-$\mu$m filter, and the filtrate is analyzed by liquid chromatography analysis. The reagents are used as standards and analyzed under the same conditions. Calibration curves are prepared based on the peak area, and the NAD concentration in the sample is calculated. The analysis conditions can be set as follows.

<Liquid chromatographic conditions>

- Column: Wakosil-II 5C18 RS (4.6 mm × 150 mm)
- Mobile phase: 0.5% ammonium phosphate buffer (pH 3.2)
- Flow rate: 1.0 mL/min
- Column temperature: 40°C
- UV detection wavelength: 210 nm

In the present invention, a yeast extract containing, in addition to 15% by mass or more of glutathione, 0.05% by mass or more of adenine, 0.05% by mass or more of adenosine, 0.010% by mass or more of folic acid and 0.010% by mass or more of riboflavin is used. There are no restrictions on the yeast extract to be used as long as it has these contents. For example, two or more yeast extracts may be mixed to prepare a yeast extract having a glutathione content of 15% by mass or more, an adenine content of 0.05% by mass or more, an adenosine content of 0.05% by mass or more, a folic acid content of 0.010 mass% or more, and a riboflavin content of 0.010 mass% or more. More desirably, the yeast extract is a yeast extract containing 15% by mass or more of glutathione, 0.1% by mass or more of adenine, 0.1% by mass or more of adenosine, 0.010% by mass or more of folic acid, and 0.010% by mass or more of riboflavin.

Since the active ingredient having the diabetes prevention effect of the present invention is a yeast extract, the yeast extract can be ingested as it is as a diabetes preventive agent or as an agent for use in the prevention or treatment of diabetes by suppressing an increase in blood sugar level, and may also be ingested as a mixture with other materials.

The agent for suppressing an increase in blood sugar level of the present invention contains the above-mentioned yeast extract as an active ingredient and can be used as a food material or as a pharmaceutical composition. Specifically, it refers to an agent for suppressing an increase in blood sugar level which is ingested orally as a food, a drink, a favorite article, a supplement, a pharmaceutical product, or the like. The form of the agent for suppressing an increase in blood sugar level is not particularly limited, and may take the form of an ordinary food or drink, as well as a tablet, a capsule, a soft capsule or a nutritional drink form.

The amount of the above-described composition to be blended in a food or drink is not particularly limited as long as the composition has the effect of suppressing an increase in blood sugar level. For example, 10 $\mu$g to 20 g of the yeast extract of the present invention may be contained per 100 g of the weight of the food or drink. Especially, the range of 100 $\mu$g to 2 g is preferable, and the range of 1 mg to 1 g is more preferable.

The amount of the composition to be blended in the pharmaceutical product is not particularly limited. For example, 10 $\mu$g to 20 g of the yeast extract of the present invention may be contained per 100 g of the weight of the pharmaceutical composition. Especially, the range of 100 $\mu$g to 2 g is preferable, and the range of 1 mg to 1 g is more preferable.

Examples

Hereinafter, the present invention will be described in more detail with reference to examples, but the present invention is not limited to the following examples.

Test 1: Measurement of glucose uptake activity in rat myoblasts (L6 cells)

L6 cells were seeded ($1 \times 10^4$ cells/well) in a 96-well plate with a D-MEM medium (Dulbecco/Vogt modified Eagle's minimal essential medium) containing 10% fetal bovine serum, and pre-cultured for 48 hours (5% $CO_2$, 37°C). Then, the medium was replaced with a D-MEM medium containing 2% horse serum and culture was performed for 96 hours (5% $CO_2$, 37°C) to induce differentiation. Subsequently, the medium was replaced with a serum-free D-MEM medium, and synchronous culture was performed for 4 hours (5% $CO_2$, 37°C) . Thereafter, the wells were washed with a KRPH buffer (Krebs Ringer Phosphate Hepes buffer), and 100 $\mu$L of a sample solution (final concentration 1 mg/mL or 5 mg/mL) dissolved in a KRPH buffer containing 100 $\mu$M 2-NBDG (2-deoxy-2-[(7-nitro-2,1,3-benzoxadiazol-4-yl)amino] -D-glucose) was added and incubated for 20 minutes (5% $CO_2$, 37°C). A solution containing no yeast extract was used as a control. Thereafter, the solution was exchanged with 100 $\mu$L of an ice-cooled KRPH buffer, and the reaction was stopped. The fluorescence intensity of 2-NBDG, as the fluorescent glucose analogue incorporated into the cells, was measured at an excitation wavelength of 470 nm and a detection wavelength of 550 nm using a microplate reader. The glucose uptake activity was determined based on the following formula as the ratio of the fluorescence intensity (FS) in the case of making the sample solution react when the fluorescence intensity (FC) of the control was taken as 100.

$$\texttt{Glucose uptake activity (\%) = (FS/FC)} \times \texttt{100}$$

(Example 1)

As a sample of Test 1, when the glucose uptake activity of the yeast extract "HITHION EXTRACT YH-15" was measured, the activity increased in a concentration-dependent manner. The "HITHION EXTRACT YH-15" used had a glutathione content of 19.0% by mass, an adenine content of 0.2% by mass, an adenosine content of 0.1% by mass, folic acid of 0.014% by mass, and riboflavin of 0.027% by mass.

(Comparative Example 1)

As a sample of Test 1, when the glucose uptake activity of insulin (final concentration 100 nM) as a positive control was measured, the activity significantly increased.

(Comparative Example 2)

As a sample of Test 1, when the glucose uptake promoting activity of the yeast extract "NUCLEAMINE" (manufactured by KOHJIN Life Sciences Co., Ltd.) was measured, no increase in glucose uptake activity was observed. The "NUCLEAMINE" used had a glutathione content of 0.8% by mass, an adenine content of 0.1% by mass, adenosine of 0.6% by mass, folic acid of 0.001% by mass, and riboflavin of 0.010% by mass.

(Comparative Example 3)

As a sample of Test 1, when the glucose uptake promoting activity of the yeast extract "Aromild G" (manufactured by KOHJIN Life Sciences Co., Ltd.) was measured, no increase in glucose uptake activity was observed. The "Aromild G" used had a glutathione content of 0.1% by mass, an adenine content of 0.1% by mass, an adenosine content of 0.2% by mass, folic acid of 0.002% by mass, and riboflavin of 0.009% by mass.

Test 2: Measurement of glucose concentration in type 2 diabetes model (GK) rat blood

"HITHION EXTRACT YH-15" having the same composition as in Example 1 was orally administered to GK rats at a dose of 10 mg/kg/day or 50 mg/kg/day orally between the ages of 4 and 20 weeks. Blood was collected from the rat tail vein at the ages of 5, 10, 15 and 19 weeks. The collected blood was centrifuged and separated into plasma and others than plasma. The glucose concentration in the separated plasma was measured with glucose CII-test Wako (Wako Pure Chemical Industries, Ltd.), and used as the fasting blood sugar level. As a result, it was confirmed that the fasting blood sugar level of the yeast extract administration group tended to be lower than that of the control group at the age of 15 weeks, and decreased significantly at the age of 19 weeks.

The results of Test Example 1 are shown in Fig. 1, and the results of Test Example 2 are shown in Fig. 2. When compared with the control, the yeast extract of the present invention had a significant effect.

It was found that the yeast extract of the present invention promotes glucose uptake in rat myoblasts and suppresses an increase in fasting blood sugar level in GK rats. Muscle tissue is a tissue which consumes the largest amount of sugar in the body, the relationship between the glucose uptake ability into muscle tissue and type II diabetes is well known. In addition, the continuation of hyperglycemic conditions results in deterioration of insulin resistance. Diabetes develops various complications throughout the body if it becomes severe. Therefore, it is considered that the yeast extract for use of the present invention is effective for preventing diabetes and, further, various complications caused by diabetes.

## Claims

1. A yeast extract for use in the prevention or treatment of diabetes by suppressing an increase in blood sugar level, having a glutathione content of 15% by mass or more, an adenine content of 0.05% by mass or more, an adenosine content of 0.05% by mass or more, a folic acid content of 0.010% by mass or more, and a riboflavin content of 0.010% by mass or more.

2. The yeast extract for use in the prevention or treatment of diabetes by suppressing an increase in blood sugar level according to claim 1, wherein the yeast of the yeast extract is Candida utilis or Saccharomyces cerevisiae.

3. An agent for use in the prevention or treatment of diabetes by suppressing an increase in blood sugar level comprising, as an active ingredient, a yeast extract having a glutathione content of 15% by mass or more, an adenine content of 0.05% by mass or more, an adenosine content of 0.05% by mass or more, a folic acid content of 0.010% by mass or more, and a riboflavin content of 0.010%.

## Patentansprüche

1. Hefeextrakt zur Verwendung in der Verhinderung oder Behandlung von Diabetes durch das Unterdrücken eines Anstiegs des Blutzuckergehalts mit einem Glutathion-Gehalt von 15 Gew.-% oder mehr, einem Adenin-Gehalt von 0,05 Gew.-% oder mehr, einem Adenosin-Gehalt von 0,05 Gew.-% oder mehr, einem Folsäure-Gehalt von 0,010 Gew.-% oder mehr, und einem Riboflavin-Gehalt von 0,010 Gew.-% oder mehr.

2. Hefeextrakt zur Verwendung in der Verhinderung oder Behandlung von Diabetes durch das Unterdrücken eines Anstiegs des Blutzuckergehalts nach Anspruch 1, wobei die Hefe des Hefeextrakts Candida utilis oder Saccharomyces cerevisiae ist.

3. Mittel zur Verwendung in der Verhinderung oder Behandlung von Diabetes durch das Unterdrücken eines Anstiegs des Blutzuckergehalts, umfassend als aktiven Inhaltsstoff einen Hefeextrakt mit einem Glutathion-Gehalt von 15 Gew.-% oder mehr, einem Adenin-Gehalt von 0,05 Gew.-% oder mehr, einem Adenosin-Gehalt von 0,05 Gew.-% oder mehr, einem Folsäure-Gehalt von 0,010 Gew.-% oder mehr, und einem Riboflavin-Gehalt von 0,010 Gew.-% oder mehr.

## Revendications

1. Extrait de levure à utiliser dans la prévention ou le traitement du diabète par suppression d'une élévation de la glycémie, dont la teneur en glutathion est de 15% en masse ou plus, la teneur en adénine est de 0,05% en masse ou plus, la teneur en adénosine est de 0,05% en masse ou plus, la teneur en acide folique est de 0,010% en masse ou plus et la teneur en riboflavine est de 0,010% en masse ou plus.

2. Extrait de levure à utiliser dans la prévention ou le traitement du diabète par suppression d'une élévation de la glycémie selon la revendication 1, dans lequel la levure de l'extrait de levure est Candida utilis ou Saccharomyces cerevisiae.

3. Agent à utiliser dans la prévention ou le traitement du diabète par suppression d'une élévation de la glycémie comprenant, comme agent actif, un extrait de levure dont la teneur en glutathion est de 15% en masse ou plus, la teneur en adénine est de 0,05% en masse ou plus, la teneur en adénosine est de 0,05% en masse ou plus, la teneur en acide folique est de 0,010% en masse ou plus et la teneur en riboflavine est de 0,010% en masse.

[Fig. 1]

[Fig. 2]

8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011144167 A
- JP 2014118374 A
- JP 2005289847 A
- JP 2005325029 A
- JP 2015021000 A
- JP 2009291076 A

- JP 2016037485 A
- JP 2000279164 A
- JP 2006042637 A
- JP 2006042638 A
- JP 2011103789 A

**Non-patent literature cited in the description**

- *Journal of Chromatography,* 1980, vol. 194, 424-428